# EUROPÄISCHE PATENTSCHRIFT

(11) **EP 1 633 469 B1**
(45) Veröffentlichungstag und Bekanntmachung des Hinweises auf die Patenterteilung: **25.11.2009**
(21) Anmeldenummer: 04739695.7
(22) Anmeldetag: 08.06.2004
(51) Int. Cl.: B01J 13/02, B01J 13/04, C11D 3/50

(54) **VERFAHREN ZUR STABILISIERUNG VON PEROXYCARBONSÄUREN IN TENSIDHALTIGEN DISPERSIONEN**
METHOD FOR STABILISING PEROXYCARBOXYLIC ACIDS IN DISPERSIONS CONTAINING SURFACTANTS
PROCEDE PERMETTANT DE STABILISER DES ACIDES PEROXYCARBOXYLIQUES DANS DES DISPERSIONS CONTENANT DES AGENTS DE SURFACE

(30) Priorität: 13.06.2003 DE 10327127; 22.12.2003 DE 10361081
(43) Veröffentlichungstag der Anmeldung: 15.03.2006
(73) Patentinhaber: Henkel AG & Co. KGaA, 40589 Düsseldorf (DE)
(72) Erfinder: SCHMIEDEL, Peter, 40599 Düsseldorf (DE); VÖLKEL, Heinz-Jürgen, 40764 Langenfeld (DE); PLANTENBERG, Thomas, 40822 Mettmann (DE)
(86) Internationale Anmeldenummer: PCT/EP2004/006166
(87) Internationale Veröffentlichungsnummer: WO 2004/110610

(56) Entgegenhaltungen:
- EP-A- 0 435 379
- EP-A- 0 653 485

## Beschreibung

Die vorliegende Erfindung betrifft ein Verfahren zur Stabilisierung von bei Raumtemperatur festen Peroxycarbonsäuren, insbesondere Imidoperoxycarbonsäuren, in tensidhaltigen, vorzugsweise wäßrigen Dispersionen, sowie die auf diese Weise erhaltenen tensidhaltigen Dispersionen und ihre Verwendung in Wasch- und Reinigungsmitteln, Zahnpflegemitteln, Haarfärbemitteln und Entfärbungs- bzw. Bleichmittelzusammensetzungen insbesondere für technische Anwendungen. Des weiteren betrifft die vorliegende Erfindung Wasch- und Reinigungsmittel, Zahnpflegemittel, Haarfärbemittel und Entfärbungs- bzw. Bleichmittelzusammensetzungen insbesondere für technische Anwendungen, welche die erfindungsgemäßen, stabilisierten tensidhaltigen Dispersionen von Peroxycarbonsäuren enthalten.

Bei flüssigen, insbesondere wasserhaltigen Wasch- und Reinigungsmitteln, die sich aufgrund ihrer positiven Produkteigenschaften, wie einer besseren und schnelleren Löslichkeit und Anwendbarkeit, einer zunehmenden Beliebtheit erfreuen, ist die Einformulierung bzw. Inkorporierung von Bleich-(mittel)komponenten aus zahlreichen Gründen problematisch. So verlieren eingesetzte Bleichmittel aufgrund von Zersetzungs- bzw. Hydrolysereaktionen und Inkompatibilitäten gegenüber anderen Bestandteilen der Waschmittelformulierung, wie z. B. Enzymen oder Tensiden, häufig schon bei der Lagerung oder aber im Rahmen der Anwendung der Produkte ihrer Aktivität. Als nachteilige Folge verliert die Waschmittelformulierung hierdurch deutlich an Waschleistung, insbesondere Bleichvermögen, so daß insbesondere bleichbare Verschmutzungen nicht mehr zufriedenstellend entfernt werden können.

Die üblicherweise für feste Waschmittelformulierungen verwendeten Bleichkomponenten, wie beispielsweise Perborate oder Percarbonate, sind feuchtigkeitsempfindlich, so daß sie in einem flüssigen und insbesondere wasserhaltigem Wasch- oder Reinigungsmittel aufgrund des Verlustes von Aktivsauerstoff häufig innerhalb weniger Tage ihre Bleichwirkung verlieren. Demgegenüber sind Peroxycarbonsäuren, insbesondere Imidoperoxycarbonsäuren, deren wichtigster Phthalimidoperoxycapronsäure (PAP) ist, effizienter und weniger hydrolyseempfindlich und im Stand der Technik als Bleichmittel für Wasch- und Reinigungsmittel bekannt. Jedoch ist ihre Lagerstabilität bei weitem nicht ausreichend, um eine langfristige Einsetzbarkeit des entsprechenden Wasch- oder Reinigungsmittels ohne einhergehenden Aktivitätsverlust zu gewährleisten. Besonders problematisch ist daher der Einsatz von Peroxycarbonsäuren, insbesondere Imidoperoxycarbonsäuren, in flüssigen Wasch- und Reinigungsmitteln.

Aufgrund der Nachteile, die sich in bezug auf eine Veränderung der Wasch- oder Reinigungsmittelformulierung infolge des Abbaus von Imidoperoxycarbonsäuren, insbesondere PAP, ergeben, wurde im Stand der Technik versucht, Imidoperoxycarbonsäuren (z. B. PAP) enthaltende Wasch- oder Reinigungsformulierungen derart zu modifizieren, daß die Imidoperoxycarbonsäure in diesen Formulierungen eine höhere Stabilität bzw. Lagerstabilität aufweist.

Daher wurde im Stand der Technik darauf abgezielt, zur Stabilisierung dieser Peroxycarbonsäuren eine schützende Hüllschicht auf die Peroxycarbonsäuren aufzubringen, um einen Kontakt mit dem wäßrigen Dispersionsmilieu zu verhindern. Die aus dem Stand der Technik bekannten Hüllschichtsysteme sind jedoch oftmals in bezug auf das Dispersionsmilieu nicht ausreichend kompatibel und bewirken nicht immer die notwendige Stabilisierung. Beispielsweise können bestimmte Hüllmaterialien von dem Dispersionsmilieu im Laufe der Zeit aufgelöst werden. Andere Hüllschichtmaterialien, insbesondere Wachse mit hohen Schmelzpunkten (vgl. EP 0 510 761 B1 bzw. US-A-5 230 822), haben den Nachteil, daß sie die umhüllten bzw. verkapselten Peroxycarbonsäuren erst bei relativ hohen Temperaturen freisetzen - und dies meist unverzögert - und zudem nichtauflösbare Rückstände hinterlassen.

Andererseits wurde im Stand der Technik versucht, das Dispersionsmilieu für die Peroxycarbonsäuren derart einzustellen, daß es stabilisierende Wirkung in bezug auf die Peroxycarbonsäuren hat. Die aus dem Stand der Technik bekannten Maßnahmen reichen jedoch nicht aus, um die Peroxycarbonsäuren in ausreichendem Maße in Gegenwart von Tensiden zu stabilisieren.

So beschreibt die EP 0 334 405 B1 wäßrige Bleichmittelzusammensetzungen mit festen, teilchenförmigen, im wesentlichen im Wasser unlöslichen organischen Peroxycarbonsäuren, wobei zur Stabilisierung der Peroxycarbonsäuren gegen Phasentrennung von der wäßrigen Flüssigkeit 1 bis 30 Ges.-% eines sekundären C₈-C₂₂-Alkansulfonats und 0,5 bis 10 Gew.-% einer Fettsäure zugegeben sind. Aufgrund der sehr speziellen Zusammensetzung der Zusätze ist eine derartige Bleichmittelzusammensetzung nicht allgemein anwendbar. Zum-anderen ist der hiermit erreichte stabilisierende Effekt nicht immer ausreichend.

In vergleichbarer Weise wird auch entsprechend der EP 0 334 404 B1 versucht, eine Stabilisierung der Peroxycarbonsäure gegen Phasentrennung von der wäßrigen Flüssigkeit zu erreichen. Eine Stabilisierung der Peroxycarbonsäuren gegen Abbau wird jedoch nicht in ausreichendem Maße gewährleistet.

Insgesamt sind im Stand der Technik keine effizienten Maßnahmen offenbart, um in wäßrigen Dispersionen befindliche Peroxycarbonsäuren ausreichend zu stabilisieren.

Vor diesem Hintergrund besteht somit eine Aufgabe der vorliegenden Erfindung darin, tensidhaltige Dispersionen von Peroxycarbonsäuren, insbesondere Imidoperoxycarbonsäuren, insbesondere Phthalimidoperoxycapronsäure (PAP), bereitzustellen, die eine hohe Lagerstabilität aufweisen, sowie ein entsprechendes Herstellungsverfahren für diese Dispersionen anzugeben.

Eine weitere Aufgabe besteht darin, lagerstabile tensidhaltige Dispersionen von Peroxycarbonsäuren, insbesondere Imidoperoxycarbonsäure, wie Phthalimidoperoxycapronsäure (PAP), mit gegenüber dem Stand der Technik verbesserten Eigenschaften sowie ein entsprechendes Herstellungsverfahren für diese Dispersionen bereitzustellen.

Noch eine weitere Aufgabe der vorliegenden Erfindung ist die Bereitstellung von tensidhaltigen Dispersionen, die Peroxycarbonsäuren in Form fester Teilchen aufweisen und zu einer guten Stabilisierung der Peroxycarbonsäuren und damit zu einer verbesserten Lagerstabilität führen. Insbesondere sollen im Rahmen der vorliegenden Erfindung Dispersionen bereitgestellt werden, die unter anderem als bzw. für Wasch- oder Reinigungsmittel, Zahnpflegemittel, Haarfärbemittel, Entfärbungs- bzw. Bleichmittelzusammensetzungen insbesondere für technische Anwendungen oder dergleichen verwendet werden können bzw. unter anderem in Wasch- oder Reinigungsmitteln, Zahnpflegemitteln, Haarfärbemitteln, Entfärbungs- bzw. Bleichmittelzusammensetzungen insbesondere für technische Anwendungen oder dergleichen stabil eingearbeitet werden können. Dabei sollen die in den Dispersionen vorhandenen Peroxycarbonsäuren im Zustand der konzentrierten Dispersion einerseits über eine hohe Lagerstabilität verfügen und andererseits während der Anwendung des Produktes, insbesondere bei Verdünnung mit Wasser etc. (z. B. während des Waschvorganges), über eine hohe Wirkleistung verfügen bzw. die volle Bleichwirkung entfalten.

In diesem Zusammenhang kann häufig beobachtet werden, daß Peroxycarbonsäuren, insbesondere PAP, in flüssigen, insbesondere wäßrigen Medien in Gegenwart von Tensiden, wie sie beispielsweise in Wasch- und Reinigungsmittelzusammensetzungen oftmals in großen Mengen z. B. von 0,5 bis 30 Gew.-%, insbesondere 5 bis 30 Gew.%, eingesetzt werden, schnell zersetzt werden, so daß ihr Einsatz in tensidhaltigen flüssigen, insbesondere wäßrigen Medien bislang nur sehr beschränkt möglich ist.

Die Anmelderin hat nun überraschenderweise herausgefunden, daß organische Peroxycarbonsäuren, wie Imidoperoxycarbonsäuren (z. B. PAP), mit einer hohen Lagerstabilität in tensidhaltige Medien bzw. Dispersionen eingearbeitet werden können, wenn diese über spezifische, stabilisierend wirkende Eigenschaften verfügen, wie sie im einzelnen nachfolgend angeführt werden.

Gegenstand der vorliegenden Erfindung gemäß einem ersten Aspekt ist somit ein Verfahren zur Stabilisierung von bei Raumtemperatur festen Peroxycarbonsäuren, insbesondere Imidoperoxycarbonsäuren, in einer tensidhaltigen, vorzugsweise wäßrigen Dispersion, wobei die tensidhaltige Dispersion derart eingestellt wird, daß ein Abbau der in der tensidhaltigen Dispersion befindlichen Peroxycarbonsäuren im Zustand der Dispersion verhindert oder aber zumindest verringert oder verlangsamt wird bzw. die Löslichkeit der Peroxycarbonsäuren in der Dispersion herabgesetzt wird. Das genane Verfahren wird in vorliegenden Anspruch 20 beschrieben.

Dabei wird erfindungsgemäß unter dem Begriff "tensidhaltige Dispersion" insbesondere ein flüssiges, insbesondere wäßriges System bzw. Medium verstanden, welches einen signifikanten Gehalt an Tensiden aufweist (z. B. in Mengen von 0,5 bis 30 Gew.-%, insbesondere 5 bis 30 Gew.-%, bezogen auf die Dispersion bzw. kontinuierliche Phase der Dispersion), wie es z. B. bei Wasch- und Reinigungsmitteln gefordert ist. Insbesondere wird unter dem Begriff "tensidhaltige Dispersion" eine derartige Zusammensetzung verstanden, die im Rahmen ihrer Anwendung über einen ausreichenden Gehalt an Tensiden verfügt, so daß z. B. eine wasch- bzw. reinigungsaktive Wirkung der Zusammensetzung vorliegt.

Das erfindungsgemäße Verfahren ermöglicht es, Peroxycarbonsäuren (z. B. PAP) in flüssigen, insbesondere wäßrigen Dispersionen bzw. Medien in Gegenwart von Tensiden effizient zu stabilisieren bzw. ihren Abbau in solchen Medien effizient zu minimieren; dies ermöglicht ihren Einsatz in derartigen Systemen. Denn ohne die entsprechenden Maßnahmen sind Peroxycarbonsäuren (z. B. PAP) in flüssigen, insbesondere wäßrigen Dispersionen bzw. Medien nicht stabil und werden rasch zersetzt, so daß ihr Einsatz in tensidhaltigen flüssigen, insbesondere wäßrigen Medien bislang nicht möglich bzw. allenfalls sehr beschränkt möglich war.

Bei den im Rahmen der vorliegenden Erfindung eingesetzten Peroxycarbonsäuren, insbesondere Imidoperoxycarbonsäuren, handelt es sich um solche, die bei Raumtemperatur (20 °C) und bei Normal- bzw. Atmosphärendruck (101.325 Pa) in Form fester Teilchen bzw. Partikel vorliegen, d. h. teilchenförmig sind.

Im Rahmen der vorliegenden Erfindung werden unter dem Begriff "Abbau" insbesondere chemische und/oder physikalische Abbauprozesse bzw. Abbaureaktionen der Peroxycarbonsäuren, insbesondere chemische Abbauprozesse bzw. Abbaureaktionen, wie Hydrolyse, Reduktion, Oxidation, Zersetzung etc., verstanden. Derartige Reaktionen führen irreversibel zu einem Abbau bzw. zu einer Zerstörung der Peroxycarbonsäuren und damit zu einer Beeinträchtigung ihrer Einsetzbarkeit, insbesondere einer Beeinträchtigung der Bleichleistung der Dispersion derartiger Peroxycarbonsäuren.

Die Anmelderin hat nun überraschenderweise herausgefunden, daß ein Abbau von Peroxycarbonsäuren, insbesondere imidoperoxyearbonsäuren (z. B. PAP), in tensidhaltigen Dispersionen, insbesondere tensidhaltigen wäßrigen Dispersionen, effizient verhindert oder zumindest deutlich minimiert bzw. reduziert werden kann, wenn der Halogenidgehalt dieser Dispersionen minimiert wird, insbesondere diese Dispersionen zumindest im wesentlichen frei von oder zumindest arm an Halogeniden, insbesondere Chlorid und/oder Bromid, sind.

Denn-die Anmelderin hat überraschenderweise herausgefunden, daß eine hohe Halogenidkonzentration, insbesondere Chlorid- bzw. Bromidionenkonzentration, wie sie beispielsweise in herkömmlichen Wasch- und Reinigungsmitteln üblich ist, zu einem verstärkten Abbau von Peroxycarbonsäuren führt. Somit kann eine Verringerung der Halogenidkonzentration, insbesondere der Chlorid- bzw. Bromidionenkonzentration, zu einem verminderten Abbau der Peroxycarbonsäure in der (konzentrierten) Dispersion führen. Folglich führt eine Reduzierung bzw. Minimierung der Halogenidkonzentration zu einem drastischen Rückgang des Abbaus bzw. zu einer deutlichen Stabilisierung der in Dispersion befindlichen, festen teilchenförmigen Peroxycarbonsäuren.

Eine gute Stabilisierung der in Dispersion befindlichen Peroxycarbonsäuren wird insbesondere dann erreicht, wenn der Gesamtgehalt an Halogenidionen, insbesondere Chlorid und/oder Bromid, bezogen auf die kontinuierliche Phase der Dispersion, 100 ppm, insbesondere 50 ppm, vorzugsweise 30 ppm, besonders bevorzugt 15 ppm, nicht übersteigt (d. h. also die gewichtsbezogene Gesamtmenge an Halogeniden in der kontinuierliche Phase der Dispersion CHalogenid (total) ≤ 100 ppm, insbesondere ≤ 50 ppm, vorzugsweise ≤ 30 ppm, besonders bevorzugt ≤ 15 ppm, beträgt). Die vorgenannten Mengen werden erfindungsgemäß als im wesentlichen halogenidfreie bzw. halogenidarme Bereiche oder Mengen verstanden. Erfindungsgemäß wird unter dem Begriff "kontinuierliche Phase der Dispersion" das Dispersionsmittel mit den darin gelösten Bestandteilen oder Inhaltstoffen (z. B. Salze, Tenside etc.) verstanden. Erfindungsgemäß bevorzugt ist das Dispersionsmittel Wasser.

Die im Rahmen der vorliegenden Erfindung durchgeführte Minimierung des Halogenidionengehalts, insbesondere des Chlorid- bzw. Bromidgehalts, in der erfindungsgemäßen Dispersion kann dabei durch Auswahl bzw. Vermeidung bestimmter Inhaltsstoffe, Bestandteile etc. erreicht werden (z. B. Verwendung von im wesentlichen halogenidfreien Komponenten, so z. B. halogenidfreien Tensiden, halogenidfreien Phosphonaten etc.). Eine geringe Halogenid-, insbesondere Chloridionenkonzentration, kann erfindungsgemäß beispielsweise durch den Einsatz von halogenidfreien Kationtensiden, z. B. in Form von Methylsulfat-, Phosphat-, Tosylat- oder Cumolsulfonatverbindungen, erreicht werden. Dagegen sind Halogenidionen, insbesondere Chlorid bzw. Bromid, in vielen technischen Waschmittelbestandteilen in zum Teil nicht unerheblicher Konzentration enthalten, wobei insbesondere technische Qualitäten anionischer Tenside genannt werden können. Daher sollten erfindungsgemäß vorzugsweise nur solche Waschmittelbestandteile, insbesondere Tenside, eingesetzt werden, die zumindest im wesentlichen keine Halogenid- bzw. Chloridionen aufweisen, und nur solche Rohstoffe ausgewählt werden, die einen besonders niedrigen halogenid- bzw. Chloridgehalt aufweisen.

Im allgemeinen sollten alle Komponenten bzw. Bestandteile, welche in dem erfindungsgemäßen Verfahren zur Herstellung der Dispersionen verwendet werden, derart ausgewählt sein, daß sie einerseits im wesentlichen halogenidfrei oder zumindest halogenidarm sind und andererseits zumindest im wesentlichen kompatibel in bezug auf die Peroxycarbonsäuren sind, d. h. es sollten keine unerwünschten chemischen Reaktionen, wie Abbaureaktionen, insbesondere keine Reduktions-, Oxidations- oder Hydrolysereaktionen, zwischen diesen Komponenten und den Peroxycarbonsäuren und keine durch die weiteren Komponenten induzierten Reaktionen der Peroxycarbonsäure auftreten, welche zu ihrem Aktivitätsverlust, insbesondere Abbau, führen.

Die Anmelderin hat nun überraschend gefunden, daß der durch die Halogenidminimierung erreichte stabilisierende Effekt in bezug auf die in tensidhaltigen Dispersion befindlichen Peroxycarbonsäuren noch dadurch gesteigert werden kann, daß die tensidhaltige Dispersion sauer, vorzugsweise leicht bis mäßig sauer, oder allenfalls neutral eingestellt wird. Vorzugsweise wird die Dispersion auf einen pH-Wert von-höchstens 7, insbesondere einen pH-Wert im Bereich von 3,5 bis 7, vorzugsweise von 4,0 bis 6,5, besonders bevorzugt von 4,5 bis 6, ganz besonders bevorzugt von etwa 5, eingestellt.

Denn Bleichmittel auf Basis von Peroxycarbonsäuren, wie PAP, können überraschenderweise in einer sauren tensidhaltigen Umgebung wirkungsvoll stabilisiert werden, da die Peroxycarbonsäuren bei derartigen pH-Werten in dem Dispersionsmittel, insbesondere Wasser, eine nur geringe Löslichkeit aufweisen und als Kristalldispersion vorliegen, wohingegen bei neutralen oder alkalischen pH-Werten eine relativ schnelle Zersetzung von Peroxycarbonsäuren, wie PAP, aufgrund der erhöhten Löslichkeit stattfindet. Dennoch sollte der pH-Wert der Dispersion, insbesondere des Wasch- und Reinigungsmittels, nicht zu sauer eingestellt werden, um eine Degeneration bzw. Inaktivierung von gegebenenfalls in der Dispersion vorhandenen Enzymen zu vermeiden. Somit stellt der im Rahmen der vorliegenden Erfindung angegebenen pH-Wert einen vor diesem Hintergrund optimierten Bereich dar.

Die Einstellung, insbesondere Absenkung bzw. Verschiebung des pH-Wertes der Dispersion in den sauren Bereich kann im Rahmen des erfindungsgemäßen Verfahrens mit Säuren bzw. sauren Salzen durchgeführt werden. Beispiele für erfindungsgemäß geeignete Säuren bzw. saure Salze zur Einstellung des pH-Wertes sind z. B. organische Polycarbonsäuren, Bisulfate und Biphosphate. Ferner können als Komplexbildner eingesetzte Phosphonate als Phosphonsäuren eingearbeitet werden und anschließend der gewünschte pH-Wert durch Zugabe von Alkalien eingestellt werden (Einstellung *pH-Wert* Verfahren).

Weiterhin konnte die Anmelderin in überraschender Weise aufzeigen, daß der durch die zuvor genannten Maßnahmen erreichte stabilisierende Effekt in bezug auf die in Dispersion befindlichen Peroxycarbonsäuren weiter gesteigert werden kann, indem die in der Dispersion vorhandenen Tenside - wie es etwa für Wasch- und Reinigungsmittel der Fall ist - in eine inaktivierte Form überführt werden, d. h. die Dispersion sollte zumindest im wesentlichen keine Tenside in aktiver Form enthalten. Dabei sollte der Gesamtgehalt an aktiven Tensiden in der Dispersion bzw. der kontinuierlichen Phase der Dispersion weniger als 5 %, insbesondere weniger als 2,5 %, vorzugsweise weniger als 1 %, jeweils bezogen auf die Dispersion bzw die kontinuierliche Phase der Dispersion, betragen. Mit anderen Worten beträgt der Gesamtgehalt an inaktivierten Tensiden in der Dispersion mehr als 95 %, insbesondere mehr als 97,5 %, vorzugsweise mehr als 99 %, jeweils bezogen auf die Gesamttenside.

Die Anmelderin konnte in diesem Zusammenbang zeigen, daß organische Peroxycarbonsäuren, insbesondere PAP, in Gegenwart von aktiven Tensiden (d. h. sich in freier bzw. micellarer Form in der Dispersion bzw. Wasch- oder Reinigungsmittelformulierung befindliche Tensiden) schnell zersetzt werden, da die Peroxycarbonsäuren durch die Tenside verstärkt gelöst werden und in diesem gelösten Zustand äußerst instabil sind. In diesem Zusammenhang führen nichtionische Tenside bzw. Niotenside, beispielsweise auf Basis von Alkylpolyglykolethern, zu einer beschleunigten Zersetzung der Peroxycarbonsäuren. Bei dem erfindungsgemäßen Verfahren sollte die Dispersion daher ein optimiertes, vorzugsweise ein geringes nichtgeladenes Tensid (Niotensid)/geladenes Tensid-Verhältnis aufweisen. Dabei sollte der Gehalt an Alkylpolyglykolethem möglichst gering sein.

Die Inaktivierung der Tenside kann durch Zugabe von Sulfaten, besonders bevorzugt Natriumsulfat, erfolgen. Hierdurch wird insbesondere ein Aussalzen der Tenside hervorgerufen (d. h. Induzierung einer Phasentrennung in eine tensidarme, kontinuierliche Phase und eine vorzugsweise lamellare, im allgemeinen hochviskose, kristalline oder flüssigkristalline tensidreiche Phase), wobei die Tenside aus der insbesondere micellaren, aktiven Form in eine vorzugsweise lamellare, kristalline oder flüssigkristalline Form (Kristall- oder Flüssigkristallbildung) überführt werden, die in einer nahezu tensidfreien kontinuierlichen Phase dispergiert ist. Der dispergierte Tensidflüssigkristall selbst, der beispielsweise durch Zentrifugation abgetrennt werden kann, soll dabei möglichst hochviskos sein. Eine besonders gute Stabilisierung der Peroxycarbonsäuren kann erreicht werden, wenn der Gehalt an freien Tensiden in den erfindungsgemäßen Wasch- und Reinigungsformullerungen in der kontinuierlichen Phase besonders bevorzugt nicht mehr als 1 % beträgt, bezogen auf die Dispersion bzw. die kontinuierliche Phase der Dispersion.

Die Inaktivierung der Tenside kann vorzugsweise durch Einbringen von Natriumsulfat in die kontinuierliche Phase der Dispersion hervorgerufen werden. Dabei kann das Natriumsulfat in Mengen von 5 bis 30 Gew.-%, insbesondere 15 bis 30 Gew.%, vorzugsweise 20 bis 30 Gew.-%, in die Dispersion eingebracht werden. Erfindungsgemäß ist unter dem Begriff "Einbringen" insbesondere ein Auflösen, beispielsweise durch Dissoziation, bzw. ein Solubilisieren des in die Dispersion eingebrachten Natriumsulfats zu verstehen.

Die Anmelderin hat nun unerwartet gefunden, daß eine besonders gute Stabilisierung der in Dispersion befindlichen Peroxycarbonsäuren dann erreicht wird, wenn sämtliche der vorgenannten Maßnahmen in der Dispersion realisiert sind (d. h. Minimierung des Halogenidgehaltes, Absenkung des pH-Wertes, Inaktivierung der Tenside, optimierter bzw. minimierter Niotensidgehalt). Die vorgenannten Maßnahmen wirken überraschend synergistisch, was sich in einer besonders effizienten Stabilisierung der dispergierten, festen teilchenförmigen Peroxycarbonsäuren zeigt, einhergehend mit einer guten Lagerstabilität derartiger Dispersionen.

Das erfindungsgemäße Verfahren zur Stabilisierung von bei Raumtemperatur festen, teilchenförmigen Peroxycarbonsäuren, insbesondere Imidoperoxycarbonsäuren, in tensidhaltigen Dispersionen, vorzugsweise wäßrigen Dispersionen, kann also insgesamt auf die Weise durchgeführt werden, daß die Dispersionen derart eingestellt werden,
- daß der Gesamtgehalt an Halogenidionen, insbesondere Chlorid und/oder Bromid, bezogen auf die kontinuierliche Phase der Dispersion, 100 ppm, insbesondere 50 ppm, vorzugsweise 30 ppm, besonders bevorzugt 15 ppm, nicht übersteigt; und/oder
- daß die Dispersionen einen pH-Wert von höchstens 7, insbesondere einen pH-Wert im Bereich von 3,5 bis 7, vorzugsweise von 4,0 bis 6,5,-besonders bevorzugt von 4,5 bis 6, ganz besonders bevorzugt von etwa 5, aufweisen; und/oder
- daß die Dispersionen zumindest im wesentlichen keine Tenside in aktiver Form enthält, insbesondere wobei der Gesamtgehalt an aktiven Tensiden in der kontinuierlichen Phase der Dispersionen weniger als 5 %, insbesondere weniger 2,5 %, vorzugsweise weniger als 1%, jeweils bezogen auf die kontinuierliche Phase der Dispersion, beträgt.

Wie die Anmelderin Unerwartet gefunden hat, kann die Stabilisierung der Peroxycarbonsäuren in den erfindungsgemäßen Dispersionen weiter gesteigert werden, indem den wäßrigen Dispersionen mindestens ein Komplexbildner, vorzugsweise in Mengen von 0 bis 10 Gew.%, zugegeben wird. Dabei kann der Komplexbildner ausgewählt sein aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacylcloheptandiphosphonat (AHP), Nitrilotriessigsäure (NTA), Citrat und/oder kurzkettigen Dicarbonsäuren. Diese Komplexbildner werden im Rahmen des erfindungsgemäßen Verfahrens insbesondere zur Komplexierung von Schwermetallionen eingesetzt, die insbesondere als Katalysatoren von Abbauprozessen fungieren und somit zu einem Abbau von Peroxycarbonsäuren, wie PAP, führen können und die beispielsweise über Wasserleitungen oder metallische Bauteile der Produktionsanlagen oder über Rohstoffe in die erfindungsgemäße tensidhaltige Dispersion (z. B. in ein Wasch- oder Reinigungsmittel) eingetragen werden können.

Weiterhin kann im Rahmen des erfindungsgemäßen Verfahrens zur weiteren Stabilitätserhöhung der Peroxycarbonsäuren der wäßrigen Dispersion mindestens eine Katalase zugegeben werden. Dabei dient die Katalase insbesondere zur Entfernung von in der Dispersion gegebenenfalls gebildetem bzw. vorhandenem Wasserstoffperoxid. Wasserstoffperoxid kann in der Dispersion gegebenenfalls durch Reaktion der Peroxycarbonsäure mit Wasser entstehen; die Zugabe einer Katalase führt dabei zu einer wirkungsvollen Verringerung des Gehalts an Wasserstoffperoxid in der Dispersion, so daß hierdurch auch oxidationsempfindliche, gegebenenfalls vorhandene weitere Inhaltsstoffe, beispielsweise Enzyme, wirkungsvoll geschützt werden. Zu diesem Zweck können den erfindungsgemäßen Wasch- oder Reinigungsmitteln gleichermaßen mindestens eine Peroxidase und/oder mindestens ein Antioxidans, gegebenenfalls zusätzlich zu der mindestens einen Katalase, zugegeben werden. Erfindungsgemäß bevorzugte Antioxidantien sind beispielsweise Ascorbinsäure, Tocopherol, Gallussäure oder deren Derivate.

Darüber hinaus hat die Anmelderin überraschenderweise herausgefunden, daß die Stabilität der Peroxycarbonsäuren in den erfindungsgemäßen tensidhaltigen Dispersionen dadurch gesteigert werden kann, indem der Dispersion mindestens ein insbesondere wassermischbares Lösemittel (z. B. Glycerin) zugegeben wird oder aber das wassermischbare Lösemittel das Dispersionsmittel der Dispersion ist. Dabei sollte das Lösemittel eine für die organische Peroxycarbonsäuren, insbesondere Imidoperoxycarbonsäuren, geringes Lösungsvermögen aufweisen. Vorzugsweise ist dieses Lösemittel Glycerin. Die Menge des Lösemittels (z. B. Glycerin) kann vorzugsweise mehr als 20 Gew.-%, besonders bevorzugt mehr als 30 Gew.-%, betragen, bezogen auf die Dispersion. Bei dieser lösemittelbasierten Variante sollte der Wassergehalt der Wasch- oder Reinigungsformulierung vorzugsweise bei etwa 5 Gew.-%, bezogen auf die Dispersion, liegen, wobei der Glyceringehalt 70 Gew.-% übersteigen kann. Glycerin besitzt ein geringes Lösungsvermögen für organische Peroxycarbonsäuren, insbesondere Imidoperoxycarbonsäuren, wie PAP, was zu einer Stabilisierung der Peroxycarbonsäure in den erfindungsgemäßen Dispersionen führen kann. Dennoch sollte die Menge an gegebenenfalls eingesetztem Glycerin derart sein, daß keine negativen Einflüsse auf die weiteren Inhaltsstoffe resultieren, insbesondere in bezug auf ihre Löslichkeit in der Dispersion. Im allgemeinen stellt jedoch Wasser das bevorzugte Dispersionsmittel dar.

Zur weiteren Erhöhung der Stabilität, insbesondere Lagerstabilität, der Peroxycarbonsäuren in den erfindungsgemäßen Dispersionen können die Peroxycarbonsäuren - sofern dies verfahrenstechnisch bzw. anwendungsbezogen gewünscht oder erforderlich ist - zusätzlich mit mindestens einer Hülle versehen werden oder in mindestens eine Matrix eingelagert werden, so daß z. B ein Kapselsystem mit einem Kapselkern auf Basis mindestens einer Peroxycarbonsäure resultiert. Bei dieser erfindungsgemäßen Ausführungsform wird die Lagerstabilität der Peroxycarbonsäuren dadurch erhöht, daß ein direkter Kontakt der Peroxycarbonsäuren mit der Umgebung, insbesondere mit der Dispersion bzw. dem Dispersionsmittel und den darin gelösten bzw. dispergierten Substanzen, zumindest im wesentlichen verhindert bzw. zumindest verringert wird.

Beispielsweise kann die Hülle oder Matrix mindestens ein anorganisches Salz, bevorzugt ein anorganisches Sulfat, besonders bevorzugt Natriumsulfat, umfassen oder hieraus bestehen. Bei einem derartigen Kapselsystem sollte die erfindungsgemäße Dispersion derart ausgestaltet sein, daß die Sulfathülle während der Lagerung zumindest im wesentlichen nicht abgebaut, insbesondere abgelöst bzw. aufgelöst wird, was insbesondere durch Zugabe eines die Löslichkeit der Hülle beeinflussenden Salzes, beispielsweise ein anorganisches Sulfat, besonders bevorzugt Natriumsulfat, bewerkstelligt werden kann. Eine Freisetzung der Peroxycarbonsäure sollte dann während der Anwendung, insbesondere in einer Waschflotte durch entsprechende Verdünnungseffekte mit einhergehendem Abbau der Hülle erfolgen.

Das Aufbringen der Hülle oder Matrix auf die Peroxycarbonsäure erfolgt vorteilhafterweise vor Einbringen in die Dispersion. Bei der Hülle oder Matrix kann es sich beispielsweise um ein Gel, beispielsweise auf Basis einer durch einen Stabilisator, insbesondere Gelbildner, verfestigten bzw. gelierten Ölphase, handeln. Zudem kann im Rahmen des erfindungsgemäßen Verfahrens die Hülle eine beispielsweise mehrschichtige Polyelektrolytkapselhülle sein. Weiterhin kann die Hülle oder Matrix beispielsweise anorganische Salze, insbesondere Sulfate und/oder Phosphate, anorganische Oxide, organische Polymere, insbesondere Celluloseether, Polyvinylalkohole (PVAI) und Polyvinylpyrrolidone (PVP) enthalten.

Durch-das Beschichten-der-Peroxycarbonsäure mit mindestens einer Hülle bzw. Matrix werden Kapselsysteme erhalten, die neben dem Kapselkern auf Basis der Peroxycarbonsäure eine Kapselhülle auf Basis des Hüllmaterials - wie nachfolgend angeführt - aufweisen. Dabei können sowohl der Kapselkern als auch die Kapselhülle weitere Substanzen zum gezielten Einstellen der Kapselsystemeigenschaften aufweisen, falls dies verfahrenstechnisch bzw. anwendungsbezogen erwünscht oder erforderlich ist. So kann der organischen Peroxycarbonsäure eine Substanz, welche bei einer Temperatur unterhalb von 80 °C, insbesondere unterhalb von 70°C, endotherme Reaktionen mit sich selbst, insbesondere Kristallwasserabspaltungsreaktionen oder Zersetzungsreaktionen, eingehen kann, aufgebracht sein. Diese Substanz kann aber auch mit der Peroxycarbonsäure vermengt bzw. vermischt sein. Beispielsweise kann diese Substanz Borsäure sein. Darüber hinaus kann die Hülle bzw. Matrix mindestens einen Komplexbildner, insbesondere wie zuvor definiert, enthalten.

Weiterhin kann durch das Aufbringen einer Hülle bzw. Matrix auf die Peroxycarbonsäure die Auflösegeschwindigkeit des Kapselsystems und damit die Freisetzung der Peroxycarbonsäuren bei der Anwendung, insbesondere in einer Waschflotte, in gewünschter Weise eingestellt werden. Somit kann ein "Controlled-release-Effekt" bezüglich der in dem erfindungsgemäßen Kapselsystem enthaltenen Peroxycarbonsäuren erzielt werden. Unter einem "Controlled-release-Effekt" ist insbesondere eine geringfügige, vorzugsweise zwischen 1 und 15 Minuten betragende Verzögerung der Auflösung des Kapselsystems bei seiner Anwendung, z. B. bei Verdünnung in einer Wasch- und Reinigungsflotte, bzw. eine Freisetzung der Peroxycarbonsäure aus dem Kapselsystem zu verstehen.

Der Abbau, insbesondere das Ab- bzw. Auflösen, der Kapselhüllen kann bei der Anwendung der Dispersion (beispielsweise in einer Waschflotte) im allgemeinen aufgrund von physikalischen bzw. chemischen Wechselwirkungen oder Reaktionen, beispielsweise Solubilisierung bzw. Dissoziationsvorgängen, insbesondere als Folge von Verdünnungseffekten in der Waschflotte, erfolgen.

Bei dem erfindungsgemäßen Verfahren kann die Teilchengröße der in die Dispersion eingebrachten organischen Peroxycarbonsäuren ≤ 3.000 µm, insbesondere ≤ 2.500 µm, vorzugsweise ≤ 2.250 µm, bevorzugt ≤ 2.000 µm, besonders bevorzugt ≤ 1.500 µm, sein. In diesem Zusammenhang sollte die Teilchengröße der organischen Peroxycarbonsäuren 10 bis 3.000 µm, insbesondere 50 bis 2.500 µm, vorzugsweise 100 bis 1.500 µm, betragen.

Der Gehalt an organischen Peroxycarbonsäuren, insbesondere Imidoperoxycarbonsäuren, bezogen auf die Dispersion, kann bei dem erfindungsgemäßen Verfahren 0,1 bis 30 Gew.-%, insbesondere 0,5 bis 25 Gew.-%, vorzugsweise 1 bis 20 Gew.-%, bevorzugt 1 bis 15 Gew.-%, betragen. Dabei kann erfindungsgemäß ein Einstellen der Teilchengröße der festen Peroxycarbonsäure beispielsweise vor dem Einbringen der Peroxycarbonsäureteilchen in die Dispersion durch den Fachmann als solche bekannte Verfahren durchgeführt werden, beispielsweise mittels Scherung, Vibration und/oder Ultraschalleintrag, Zerkleinern, Vermahlen etc., so daß erfindungsgemäß eine gezielte Anpassung der Teilchengröße entsprechend ihrer jeweiligen späteren Verwendung möglich ist.

Durch die Auswahl der Teilchengröße sowie des Gehalts an anorganischen Peroxycarbonsäuren in den Dispersionen kann eine gezielte Einstellung bzw. Anpassung in bezug auf die gewünschten Produkteigenschaften vorgenommen werden.

Bei dem erfindungsgemäßen Verfahren werden als in der erfindungsgemäßen Dispersion zu stabilisierende Substanzen organische Peroxycarbonsäuren verwendet. Letztere können aus organischen Mono- und Diperoxycarbonsäuren ausgewählt sein. Beispiele sind insbesondere Dodecandiperoxysäure oder vorzugsweise Imidoperoxycarbonsäuren, besonders bevorzugt 6-Phthalimidoperoxycapronsäuren (6-Phthalimidoperoxyhexansäure, PAP). Dabei sollte die Peroxycarbonsäure vorteilhafterweise bei Atmosphärendruck (101.325 Pa) einen Schmelzpunkt oberhalb von 20 °C, insbesondere oberhalb von 25 °C, vorzugsweise oberhalb von 35 °C, bevorzugt oberhalb von 45 °C, besonders bevorzugt oberhalb von 50 °C, ganz besonders bevorzugt oberhalb von 100 °C, aufweisen; hierdurch ist gewährleistet, daß die verwendete Peroxycarbonsäure im wesentlichen in Form fester Teilchen vorliegt, so daß ein Abbau der Peroxycarbonsäure in der erfindungsgemäßen Dispersion zumindest verringert ist.

Ein weiterer Gegenstand - gemäß einem zweiten Aspekt der vorliegenden Erfindung - sind die nach dem erfindungsgemäßen Verfahren herstellbaren, lagerstabilen tensidhaltigen Dispersionen, insbesondere tensidhaltige wäßrige Dispersionen, von bei Raumtemperatur festen Peroxycarbonsäuren, insbesondere Imidoperoxycarbonsäuren. Dabei ist die erfindungsgemäße tensidhaltige Dispersion derart eingestellt, daß ein Abbau der in der Dispersion befindlichen Peroxycarbonsäuren im Zustand der tensidhaltigen Dispersion verhindert oder aber zumindest verringert oder verlangsamt ist bzw. daß die Löslichkeit der Peroxycarbonsäuren in der tensidhaltigen Dispersion herabgesetzt ist.

Eine erfindungsgemäße, lagerstabile tensidhaltige Dispersion kann dabei derart eingestellt sein,
- daß der Gehalt an Halogenidionen, insbesondere Chlorid und/oder Bromid, bezogen auf die kontinuierliche Phase der Dispersion, 100 ppm, insbesondere 50 ppm, vorzugsweise 30 ppm, besonders bevorzugt 15 ppm, nicht übersteigt; und/oder
- daß die Dispersion einen pH-Wert von höchstens 7, insbesondere einen pH-Wert im Bereich von 3,5 bis 7, vorzugsweise von 4,0 bis 6,5, besonders bevorzugt von 4,5 bis 6, ganz besonders bevorzugt von etwa 5, aufweist; und/oder
- daß die Dispersion zumindest im wesentlichen keine Tenside in aktiver Form enthält, insbesondere wobei der Gesamtgehalt an aktiven Tensiden in der kontinuierlichen Phase der Dispersion weniger als 5 %, insbesondere weniger als 2,5 %, vorzugsweise weniger als 1 %, jeweils bezogen auf die kontinuierliche Phase der Dispersion, beträgt.

Die genane dispersion wird in den vorliegenden Ansprüchen 1-6 beschrieben.

Für weitere Einzelheiten zu den erfindungsgemäßen Dispersionen kann auf obige Ausführungen zu dem erfindungsgemäßen Verfahren verwiesen werden, die für die erfindungsgemäßen Dispersionen entsprechend gelten.

Die erfindungsgemäßen Dispersionen weisen zahlreiche Verwendungsmöglichkeiten auf, so können die erfindungsgemäßen Dispersionen - gemäß einem weiteren Aspekt der vorliegenden Erfindung - in oder. als Wasch- oder Reinigungsmittel(n), insbesondere flüssige(n) Wasch- und Reinigungsmittelzusammensetzungen, Zahnpflegemittel(n), Haarfärbemittel(n) oder Entfärbungs- bzw. Bleichmittelzusammensetzungen für technische Anwendungen eingesetzt werden.

In diesem Zusammenhang weisen die vorgenannten Formulierungen bzw. Zusammensetzungen eine hohe Lagerstabilität in bezug auf die Peroxycarbonsäuren auf, so daß sie auch nach längeren Lagerzeiten noch über eine hohe Wirksamkeit, insbesondere Bleichleistung, verfügen.

Ein weiterer Gegenstand der vorliegenden Erfindung - gemäß einem weiteren Aspekt der vorliegenden Erfindung - sind Wasch- und Reinigungsmittel, Zahnpflegemittel, Haarfärbemittel oder Entfärbungs- bzw. Bleichmittelzusammensetzungen für technische Anwendungen, welche die erfindungsgemäßen Dispersionen enthalten.

Die erfindungsgemäßen Wasch- und Reinigungsmittel können zur Reinigung harter Oberflächen und/oder weicher, insbesondere textiler Oberflächen verwendet werden. Die erfindungsgemäßen Wasch- und Reinigungsmittel können insbesondere als Geschirrspülmittel, Allzweckreiniger, Badreiniger, Fußbodenreiniger, Autoreiniger, Glasreiniger, Möbelpflegemittel bzw. -reiniger, Fassadenreiniger, Waschmittel oder dergleichen verwendet werden, besonders bevorzugt als Waschmittel. Weiterhin, sind die erfindungsgemäßen Wasch- und Reinigungsmittel vorzugsweise zur Reinigung von Fasern, Textilien, Teppichen und dergleichen geeignet.

Die erfindungsgemäßen Wasch- und Reinigungsmittel enthalten neben den erfindungsgemäßen Dispersionen an sich übliche und dem Fachmann als solche bekannte Inhaltsstoffe bzw. Bestandteile (z. B. Tenside, Duftstoffe, Farbstoffe, Enzyme, Enzymstabilisatoren, Geruchsstoffe bzw. -builder, pH-Wert-Einstellmittel, andere Bleichmittel, Bleichaktivatoren, Silberschutzmittel, schmutzabweisende Substanzen, optische Aufheller, Vergrauungsinhibitoren, Desintegrationshilfsmittel, Verdickungsmittel, Entschäumer, Komplexbildner für Schwermetalle, schmutzabweisende Substanzen bzw. Soil-repellents, Farbübertragungsinhibitoren, Lösungsmittel, optische Aufheller und/oder weitere übliche Inhaltsstoffe), wobei im Rahmen der vorliegenden Erfindung auf die Kompatiblität der einzelnen Inhaltsstoffe bzw. Bestandteile sowohl untereinander sowie im Hinblick auf die erfindungsgemäßen Dispersionen bzw. auf die darin vorhandenen Peroxycarbonsäuren geachtet werden sollte, was durch gezielte Auswahl der Inhaltsstoffe bzw. Bestandteile bzw. ihrer jeweiligen Mengenverhältnisse realisiert wird. Auf diese Weise kann eine unerwünschte Wechselwirkung der Inhaltsstoffe bzw. Bestandteile mit den in den erfindungsgemäßen Dispersionen eingelagerten Peroxycarbonsäuren vermieden werden.

Ein erfindungsgemäßes Wasch- oder Reinigungsmittel, insbesondere flüssiges Wasch- oder Reinigungsmittel, enthält beispielsweise die folgenden Inhaltsstoffe:
(i) mindestens eine feste, teilchenförmige organische Peroxycarbonsäure, insbesondere Imidoperoxycarbonsäure, in Mengen von 0,1 bis 30 Gew.-%, insbesondere 0,5 bis 25 Gew.-%, vorzugsweise 1 bis 20 Gew.-%, bevorzugt 1 bis 15 Gew.-%; und/oder
(ii) Tenside, vorzugsweise in inaktivierter Form, insbesondere kationische und/oder anionische Tenside, vorzugsweise in Mengen von 0 bis 30 Gew.-% und/oder nichtionische Tenside, vorzugsweise in Mengen von 0 bis 30 Gew.-%; und/oder
(iii) gegebenenfalls Elektrolyte, insbesondere anorganische und/oder organische Salze, insbesondere Phosphat, Citrat und/oder Sulfat, besonders bevorzugt Natriumsulfat, vorzugsweise in Mengen von 5 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettigen Dicarbonsäuren, vorzugsweise in Mengen von 0 bis 10 Gew.-%; und/oder
(v) gegebenenfalls Enzyme, wie Proteasen, Amylasen, Cellulasen und/oder Lipasen, und/oder Enzymstabilisatoren, vorzugsweise in Mengen von 0 bis 10 Gew.-%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30 °C, und/oder Citronensäure und/oder Citrat, vorzugsweise in Mengen von 0 bis 15 Gew.%; und/oder
(vii) gegebenenfalls Duftstoffe, vorzugsweise in Mengen von 0 bis 5 Gew.-%; und/oder
(viii) gegebenenfalls Hilfsstoffe, wie Entschäumer, pH-Regulatoren, Rheologiemodifikatören (Verdicker), Lösungsmittel, Farbstoffe; und/oder
(ix) gegebenenfalls weitere übliche Inhaltsstoffe, wie Aufheller; und/oder
(x) Wasser;
wobei sämtliche Gewichtsangaben auf das Wasch- bzw. Reinigungsmittel bezogen sind.

Im allgemeinen sollte die erfindungsgemäße Wasch- oder Reinigungsmittelformulierung derart gestaltet sein, daß die Stabilität der Peroxycarbonsäuren zumindest im wesentlichen nicht verringert wird. Daher sollten die Komponenten, welche in dem erfindungsgemäßen Wasch- oder Reinigungsmittel eingesetzt werden, derart ausgewählt sein, daß sie zumindest im wesentlichen kompatibel in bezug auf die Peroxycarbonsäuren sind, d. h. es sollten insbesondere in dem Wasch- oder Reinigungsmittel selbst, insbesondere in dem Zeitraum vor seiner Anwendung (Lagerzeit), keine unerwünschten chemischen Reaktionen, wie insbesondere Abbau-, Oxidations- bzw. Reduktionsreaktionen und/oder Hydrolysereaktionen, zwischen diesen Komponenten und den Peroxycarbonsäuren auftreten, welche zu einem vorzeitigen Abbau und zu einem Aktivitätsverlust der Peroxycarbonsäuren führen.

Bei den erfindungsgemäßen Wasch- oder Reinigungsmitteln, insbesondere flüssigen Wasch- oder Reinigungsmitteln, sollten die Tenside in der Wasch- oder Reinigungsmittelformulierung inaktiviert sein, insbesondere durch Aussalzen, d. h. Induzierung einer Phasentrennung in eine tensidarme, kontinuierliche Phase und eine vorzugsweise lamellare, im allgemeinen hochviskose, kristalline oder flüssigkristalline tensidreiche Phase, vorzugsweise durch Einbringen einer Sulfatverbindung, besonders bevorzugt Natriumsulfat, in die Wasch- oder Reinigungsmittelformulierung.

Wie oben geschildert, führt die Inaktivierung der Tenside zu einem effektiven Schutz bzw. zu einer Erhöhung der Stabilität der Peroxycarbonsäuren. Der Gehalt an freien Tensiden in den erfindungsgemäßen Wach- und Reinigungsmittelformulierungen sollte in der kontinuierlichen Phase bevorzugt nicht mehr als 1 % betragen. In diesem Zusammenhang sollte auch in dem erfindungsgemäßen Wasch- oder Reinigungsmittel - entsprechend den Ausführungen zu der erfindungsgemäßen Dispersion bzw. dem Herstellungsverfahren - ein optimiertes bzw. möglichst kleines Niotensid/geladenes Tensid-Verhältnis vorliegen. Dabei sollte der Gehalt an Alkylpolyglykolethern möglichst gering sein.

Weiterhin sollte der Gehalt an anorganischem Salz, besonders bevorzugt Natriumsulfat, in dem Wasch- oder Reinigungsmittel derart gewählt sein, daß die Tenside in dem Wasch- oder Reinigungsmittel zumindest im wesentlichen inaktiviert sind, insbesondere durch Aussalzen, vorzugsweise durch Einbringen mindestens einer Sulfatverbindung, besonders bevorzugt Natriumsulfat. Dabei sollte die Sulfatkonzentration in dem erfindungsgemäßen Wasch- oder Reinigungsmittel derart gewählt sein, daß bei der Anwendung des Wasch- oder Reinigungsmittels in der Waschflotte die Tenside wieder in aktiver Form vorliegen, was beispielsweise durch einen Verdünnungseffekt beim Eintragen des Wasch- oder Reinigungsmittels in die Waschflotte erreicht werden kann. Insbesondere soll die Konzentration so gewählt sein, daß - wie oben erwähnt - in der kontinuierlichen Phase des Wasch- oder Reinigungsmittels insbesondere weniger als 1 % aktives Tensid vorliegt und daß bei Temperaturabsenkung, insbesondere bei Temperaturabsenkungen bis auf 0 °C, keine Auskristallisation des Sulfats stattfindet.

Die erfindungsgemäßen Wasch- und Reinigungsmittel sollten zumindest im wesentlichen keinen erhöhten Chlorid- bzw. Bromidionengehalt aufweisen, was durch den Einsatz von Methylsulfat-, Phosphat-, Tosylat- oder Cumolsulfonatverbindung erreich werden kann. Ferner sollten Rohstoffe ausgewählt werden, die einen besonders niedrigen Chlorid- bzw. Bromidgehalt aufweisen.

Die erfindungsgemäßen Wasch- oder Reinigungsmittel können mindestens eine Fettsäure enthalten. Dabei sind gesättigte und/oder verzweigte Fettsäuren, insbesondere mit einem Schmelzpunkt unterhalb von 30°C, erfindungsgemäß bevorzugt. Im Rahmen der vorliegenden Erfindung kann beispielsweise Isocarb-16^{®} der Firma Sasol in den erfindungsgemäßen Wasch- oder Reinigungsmitteln eingesetzt werden.

Für weitere Einzelheiten zu den erfindungsgemäßen Wasch- und Reinigungsmitteln kann auf obige Ausführungen zu dem erfindungsgemäßen Verfahren und den erfindungsgemäßen Dispersionen verwiesen werden.

Um in der Waschflotte eine ausreichende Bleichleistung zu erzielen, sollte das erfindungsgemäße Wasch- oder Reinigungsmittel bzw. die erfindungsgemäßen Dispersionen derart konfektioniert sein, daß die Peroxycarbonsäuren während ihrer Anwendung, insbesondere in einer Waschflotte, ausreichend schnell aktiviert bzw. freigesetzt werden. Die Aktivierung bzw. Freisetzung der Peroxycarbonsäuren erfolgt dabei insbesondere durch physikalische bzw. physikochemische oder durch chemische Prozesse. So führt eine Verdünnung des anorganischen Salzes, besonders bevorzugt Natriumsulfat, in der Waschflotte zu einer Überführung der Tenside aus ihrer inaktivierten Form (beispielsweise als Flüssigkristalle vorliegende Tenside) in die aktive, micellare Form, so daß die auf diese Weise aktivierten Tenside die festen Peroxycarbonsäuren an- und/oder auflösen können. Bei der Verdünnung in der Waschflotte tritt gleichzeitig ein deutlicher pH-Wert-Sprung des im allgemeinen sauer eingestellten Wasch- und Reinigungsmittels ein, so daß auch hierdurch die Löslichkeit der Peroxycarbonsäure deutlich zunimmt.

Weiterhin sollte das Wasch- oder Reinigungsmittel derart zusammengesetzt sein, daß es einen Abbau, insbesondere ein Auf- bzw. Ablösen einer gegebenenfalls auf die Peroxycarbonsäuren aufgebrachten Hülle bzw. Matrix - insbesondere wie zuvor geschildert - während der Anwendung insbesondere in der Waschflotte gewährleistet. So kann beispielsweise ein Auflösen der gegebenenfalls vorhandenen Sulfathülle durch die zuvor angesprochenen Verdünnungseffekte erreicht werden. Weiterhin kann durch die Aktivierung der Tenside in der Waschflotte eine gegebenenfalls vorhandene Gelmatrix aufgelöst bzw. solubilisiert werden. In diesem Zusammenhang kann auch ein Ablösung, insbesondere Solubilisieren, einer gegebenenfalls Polyelektrolythülle durch die aktivierten Tenside unterstützt bzw. hervorgerufen werden. Auch mechanische Kräfte spielen hier eine Rolle.

Die vorliegende Erfindung weist gegenüber dem Stand der Technik eine Reihe von Vorteilen auf:

Die erfindungsgemäßen Dispersionen führen zu einer deutlichen Erhöhung der Lagerstabilität der darin eingelagerten Peroxycarbonsäuren - insbesondere in Zusammenhang mit einer geringen Chlorid- bzw. Bromidionenkonzentration -, so daß die Wirksamkeit der Peroxycarbonsäuren, insbesondere Bleichleistung, auch nach einem langen Zeitraum bzw. einer langen Lagerzeit gewährleistet ist. Dabei führen die jeweiligen Maßnahmen bzw. Einstellungen der Dispersion im Rahmen eines synergistischen Effektes zu einer signifikanten Erhöhung der Lagerstabilität der Peroxycarbonsäuren in der Dispersion.

Das erfindungsgemäße Verfahren zur Herstellung der Dispersionen ist einfach und gut handzuhaben, wobei auf den Einsatz aufwendig herzustellender und daher oftmals kostenintensiver Substanzen verzichtet werden kann. Daher eignet sich das Verfahren hervorragend für einen Einsatz im großtechnisch-industriellen Maßstab.

Aufgrund der gezielten Abstimmung der jeweiligen Eigenschaften der Dispersion, insbesondere Chloridgehalt, Inaktivierung der Tenside, Einstellung des pH-Wertes etc., wird einerseits eine außerordentlich gute Stabilisierung der Peroxycarbonsäuren erreicht, wobei insbesondere durch das Zusammenwirken dieser EinZelmaßnahmen ein synergistischer Effekt in bezug auf die Stabilitätserhöhung erreicht werden kann. Andererseits ist durch die erfindungsgemäßen Dispersionen bzw. Wasch- und Reinigungsmittel gewährleistet, daß die Peroxycarbonsäuren bei ihrer Anwendung, insbesondere in einer Waschflotte, freigesetzt bzw. aktiviert werden und somit eine hervorragende Waschleistung, insbesondere Bleichleistung, der entsprechenden Formulierung vorliegt.

Des weiteren können die Dispersionen in bezug auf ihre Zusammensetzung und ihren Wirkstoffgehalt weit variiert bzw. maßgeschneidert werden können, so daß eine individuelle Anpassung an die jeweiligen Anforderungen, insbesondere auf Wasch- und Reinigungsmittel, erfolgen kann. Dabei kann die Dispersion aufgrund ihrer spezifischen Ausbildung in einem großen Bereich für verschiedene Zusammensetzungen quasi universell eingesetzt werden.

Weiterhin können in die erfindungsgemäße Dispersion auch Peroxycarbonsäuren eingebracht werden, die zur weiteren Steigerung der Stabilität mit einer Hülle bzw. Matrix versehen werden, d. h. die erfindungsgemäße Dispersion ist auch in bezug auf derartige Kapselsysteme kompatibel.

Die erfindungsgemäßen Wasch- und Reinigungsmittelformulierungen weisen aufgrund ihrer zuvor angeführten, aufeinander abgestimmten und synergistisch wirkenden Anpassungen, wie insbesondere ein geringer Halogenidionengehalt, Optimierung des pH-Wertes, Zugabe von Komplexbildnern, Inaktivierung der Tenside, Einsatz spezieller Lösemittel bzw. spezieller Enzyme, wie Katalasen oder Peroxidasen, Zugabe von Antioxidantien, gegenüber dem Stand der Technik erhebliche Vorteile auf, da ein Abbau der empfindlichen Bleichmittel auf Peroxycarbonsäurebasis deutlich vermindert wird.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der folgenden Ausführungsbeispiele verdeutlicht, welche die Erfindung jedoch keinesfalls beschränken.

### Ausführungsbeispiele:

### Beispiel 1:

In diesem Beispiel wird modellhaft gezeigt, wie die Stabilität von PAP durch Chlorid beeinträchtigt wird: Es wurde eine 3%ige wäßrige Dispersion von PAP (aus Eureco^{®} W in destilliertem Wasser) mit verschiedenen Konzentrationen an NaCl versetzt und bei 40 °C eingelagert. Nach unterschiedlichen Zeitintervallen wurde der verbliebene Bruchteil an PAP (in %) bestimmt. Dieser ist in der folgenden Tabelle angegeben:

| c(NaCl) (Gew.-%) | 0 | 0,03 | 0,1 | 0,3 | 1 | 3 | 10 |
|---|---|---|---|---|---|---|---|
| 1 Tag | 100 | 98 | 97,7 | 93 | 86 | 75,7 | 48 |
| 4 Tage | 97,7 | 94 | 86,8 | 76,7 | 51,7 | 12,5 | 7,3 |

Man erkennt einen vermehrten Abbau mit zunehmendem Chloridgehalt.

### Beispiel 2:

In diesem Beispiel wird gezeigt, wie durch den Einsatz von Rohstoffen - hier technischen Tensiden - mit erfindungsgemäßem Chloridgehalt die Stabilität von PAP deutlich gesteigert werden kann:

Es wurden Lösungen folgender Zusammensetzung hergestellt:
1. Vergleichsbeispiel:
   3 % PAP
   15 % SDS, Texapon^{®} K-12 (Fa. Cognis), Chloridgehalt ~ 0,4 %
   Rest Wasser
2. erfindungsgemäß:
   3 % PAP
   15 % SDS, umkristallisiert, Chloridgehalt < 1 ppm
   Rest Wasser

Die Proben wurden bei Raumtemperatur gelagert. In der folgenden Tabelle sind die Erhaltungsgrade des PAP angegeben:

| | 3 Tage | 1 Woche |
|---|---|---|
| Vergleichsbeispiel | **-** | 57,1 |
| erfindungsgemäß | - | 93,3 |

## Patentansprüche

1. Lagerstabile tensidhaltige wäßrige Dispersion von bei Raumtemperatur festen, teilchenförmigen Peroxycarbonsäuren, insbesondere Imidoperoxycarbonsäuren, **dadurch gekennzeichnet, daß** in der Dispersion der Gesamtgehalt an Halogenidionen, insbesondere Chlorid und/oder Bromid, bezogen auf die kontinuierliche Phase der Dispersion, 100 ppm, insbesondere 50 ppm, vorzugsweise 30 ppm, besonders bevorzugt 15 ppm, nicht übersteigt; daß die Dispersion einen pH-Wert von höchstens 7, insbesondere einen pH-Wert im Bereich von 3,5 bis 7, vorzugsweise von 4,0 bis 6,5, besonders bevorzugt von 4,5 bis 6, ganz besonders bevorzugt von etwa 5, aufweist; und daß die Dispersion zumindest im wesentlichen keine Tenside in aktiver Form enthält, wobei der Gesamtgehalt an aktiven Tensiden in der kontinuierlichen Phase der Dispersion weniger als 5 %, insbesondere weniger als 2,5 %, vorzugsweise weniger als 1 %, jeweils bezogen auf die kontinuierliche Phase der Dispersion, beträgt.

2. Dispersion nach Anspruch 1, **dadurch gekennzeichnet, daß** die Dispersion mindestens einen Komplexbildner enthält und/oder daß die Dispersion mindestens eine Katalase und/oder mindestens eine Peroxidase, vorzugsweise eine Katalase, und/oder mindestens ein Antioxidans enthält und/oder daß die Dispersion mindestens ein insbesondere wassermischbares Lösemittel mit einem für die organische Peroxycarbonsäuren, insbesondere Imidoperoxycarbonsäuren, geringen Lösungsvermögen, vorzugsweise Glycerin, enthält.

3. Dispersion nach Anspruch 1 oder 2, **dadurch gekennzeichnet, daß** die Peroxycarbonsäuren zusätzlich mit mindestens einer Hülle versehen sind oder in mindestens eine Matrix eingelagert sind.

4. Dispersion nach einem der Ansprüche 1 bis 3, **dadurch gekennzeichnet, daß** die Teilchengröße der eingesetzten organischen Peroxycarbonsäuren ≤ 3.000 µm, insbesondere ≤ 2.500 µm, vorzugsweise ≤ 2.250 µm, bevorzugt ≤ 2.000 µm, besonders bevorzugt ≤ 1.500 ≤m, ist und/oder daß die Teilchengröße der eingesetzten organischen Peroxycarbonsäuren 10 bis 3.000 µm, insbesondere 50 bis 2.500 µm, vorzugsweise 100 bis 1.500 µm, beträgt.

5. Dispersion nach einem der Ansprüche 1 bis 4, **dadurch gekennzeichnet, daß** der Gehalt an organischen Peroxycarbonsäuren, insbesondere Imidoperoxycarbonsäuren, bezogen auf die Dispersion, 0,1 bis 30 Gew.-%, insbesondere 0,5 bis 25 Gew.-%, vorzugsweise 1 bis 20 Gew.-%, bevorzugt 1 bis 15 Gew.-%, beträgt.

6. Dispersion nach einem der Ansprüche 1 bis 5, **dadurch gekennzeichnet, daß** die organischen Peroxycarbonsäuren ausgewählt sind aus organischen Mono- und Diperoxycarbonsäuren, insbesondere Dodekandiperoxysäure oder vorzugsweise Imidoperoxycarbonsäuren, besonders bevorzugt 6-Phthalimidoperoxycapronsäure (6-Phthalimidoperoxyhexansäure, PAP), und/oder daß die Peroxycarbonsäuren bei Atmosphärendruck einen Schmelzpunkt oberhalb von 20°C, insbesondere oberhalb von 25 °C, vorzugsweise oberhalb von 35 °C, bevorzugt oberhalb von 45 °C, besonders bevorzugt oberhalb von 50 °C, ganz besonders bevorzugt oberhalb von 100 °C, aufweisen.

7. Verwendung der Dispersionen nach einem der Ansprüchen 1 bis 6 in oder als Wasch- und Reinigungsmittel(n), insbesondere flüssige(n) Wasch- und Reinigungsmittelzusammensetzungen, Zahnpflegemittel(n), Haarfärbemiltel(n) oder Entfärbungs- bzw. Bleichmittelzusammensetzungen für technische Anwendungen.

8. Wasch- und Reinigungsmittel, insbesondere flüssige Wasch- und Reinigungsmittelzusammensetzung, Zahnpflegemittel, Haarfärbemittel oder Entfärbungs- bzw. Bleichmittel für technische Anwendungen, enthaltend eine Dispersion nach einem der Ansprüche 1 bis 6.

9. Wasch- oder Reinigungsmittel nach Anspruch 8, enthaltend:
(i) mindestens eine organische Peroxycarbonsäure, insbesondere Imidoperoxycarbonsäure, in Mengen von 0,1 bis 30 Gew.-%, insbesondere 0,5 bis 25 Gew.-%, vorzugsweise 1 bis 20 Gew.%, bevorzugt 1 bis 15 Gew.-%; und/oder
(ii) Tenside, vorzugsweise in inaktivierter Form, insbesondere kationische und/oder anionische Tenside, vorzugsweise in Mengen von 0 bis 30 Gew.-%, und/oder nichtionische Tenside, vorzugweise in Mengen von 0 bis 30 Gew.-%; und/oder
(iii) gegebenenfalls Elektrolyte, insbesondere anorganische und/oder organische Salze, insbesondere Phosphat, Citrat und/oder Sulfat, besonders bevorzugt Natriumsulfat, vorzugsweise in Mengen von 5 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxy-ethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, vorzugsweise in Mengen von 0 bis 10 Gew.-%; und/oder
(v) gegebenenfalls Enzyme, wie Proteasen, Amylasen, Cellulasen und/oder Lipasen, und/oder Enzymstabilisatoren, vorzugsweise in Mengen von 0 bis 10 Gew.%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30°C, und/oder Citronensäure und/oder Citrat, vorzugsweise in Mengen von 0 bis 15 Gew.-%; und/oder
(vii) gegebenenfalls Duftstoffe, vorzugsweise in Mengen von 0 bis 5 Gew.-%; und/oder
(viii) gegebenenfalls Hilfsstoffe, wie Entschäumer, pH-Regulatoren, Rheologiemodifikatoren (Verdicker), Lösungsmittel, Farbstoffe, und/oder
(ix) gegebenenfalls weitere übliche Inhaltsstoffe, wie Aufheller; und/oder
(x) Wasser;
wobei sämtliche Gewichtsangaben auf das Wasch- bzw. Reinigungsmittel bezogen sind.

10. Verfahren zur Stabilisierung von bei Raumtemperatur festen Peroxycarbonsäuren, insbesondere Imidoperoxycarbonsäuren, in tensidhaltigen, insbesondere wäßrigen Dispersionen, **dadurch gekennzeichnet, daß** man
- den Gesamtgehalt an Halogenidionen, insbesondere Chlorid und/oder Bromid, bezogen auf die kontinuierliche Phase der Dispersion, auf Mengen nicht über 100 ppm, insbesondere nicht über 50 ppm, vorzugsweise nicht über 30 ppm, besonders bevorzugt nicht über 15 ppm, einstellt;
- den pH-Wert der Dispersion auf höchstens 7, insbesondere im Bereich von 3,5 bis 7, vorzugsweise von 4,0 bis 6,5, besonders bevorzugt von 4,5 bis 6, ganz besonders bevorzugt von etwa 5, einstellt; und
- den Gesamtgehalt an aktiven Tensiden in der kontinuierlichen Phase der Dispersion auf weniger als 5 %, insbesondere weniger als 2,5 %, vorzugsweise weniger als 1 %, jeweils bezogen auf die kontinuierliche Phase der Dispersion, einstellt.

## Claims

1. Storage stable aqueous surfactant-containing dispersion of particulate percarboxylic acids, particularly imidopercarboxylic acids, which are solid at room temperature, wherein the total content of halide ions, particularly chloride and/or bromide, in the dispersion, based on the continuous dispersion phase, does not exceed 100 ppm, particularly 50 ppm, preferably 30 ppm, particularly preferably 15 ppm; and/or the dispersion has a pH of maximum 7, particularly a pH of 3.5 to 7, preferably from 4.0 to 6.5, particularly preferably from 4.5 to 6, quite particularly, preferably of about 5; and/or the dispersion, at least essentially, comprises no surfactants in active form, wherein the total active surfactant content in the continuous phase of the dispersion is less than 5 %, particularly less than 2.5 %, preferably less than 1 %, based on the continuous dispersion phase.

2. Dispersion according to Claim 1, wherein the dispersion comprises at least one chelating agent and/or the dispersion comprises at least one catalase and/or at least one peroxidase, preferably a catalase, and/or at least one antioxidant and/or the dispersion comprises at least one water-miscible solvent, in which the organic polycarboxylic acid, particularly imidopercarboxylic acid is poorly soluble, preferably glycerin.

3. Dispersion according to Claim 1 or 2, wherein the percarboxylic acids are additionally provided with at least one shell or are deposited in at least one matrix.

4. Dispersion according to one of Claims 1 to 3, wherein the particle size of the added organic percarboxylic acids is ≤ 3000 µm, particularly ≤ 2500 µm, advantageously ≤ 2250 µm, preferably ≤ 2000 µm, particularly preferably ≤ 1500 µm, and/or the particle size of the added organic percarboxylic acids is 10 to 3000 µm, particularly 50 to 2500 µm, advantageously 100 to 1500 µm.

5. Dispersion according to one of Claims 1 to 4, wherein the content of organic percarboxylic acids, particularly imidopercarboxylic acids, based on the dispersion, is 0.1 to 30 wt.%, particularly 0.5 to 25 wt.%, advantageously 1 to 20 wt.%, preferably 1 to 15 wt.%.

6. Dispersion according to one of Claims 1 to 5, wherein the organic percarboxylic acids are selected from organic mono and dipercarboxylic acids, particularly dodecanebis(peroxoic) acid or preferably imidopercarboxylic acids, particularly preferably 6-phthalimidopercaproic acid (6-phthalimidoperhexanoic acid, PAP), and/or that the percarboxylic acids have a melting point at atmospheric pressure above 20 °C, particularly above 25°C, advantageously above 35 °C, preferably above 45°C, particularly preferably above 50°C; quite particularly preferably above 100°C.

7. Use of the dispersions according to Claims 1 to 6 in or as washing and cleaning agents, particularly liquid washing and cleaning agent compositions, tooth care products, hair colorants or decolorizing or bleaching agent compositions for technical uses.

8. Washing and cleaning agents, particularly liquid washing and cleaning agent compositions, tooth care products, hair colorants or decolorizing or bleaching agents for technical uses, comprising dispersions according to one of Claims 1 to 6.

9. Washing or cleaning agent according to Claim 8, comprising:
(i) at least one organic percarboxylic acid, particularly imidopercarboxylic acid, in amounts of 0.1 to 30 wt.%, particularly 0.5 to 25 wt.%, advantageously 1 to 20 wt.%, preferably 1 to 15 wt.%; and/or
(ii) surfactants, advantageously in inactivated form, particularly cationic and/or anionic surfactants, advantageously in amounts of 0 to 30 wt.%, and/or non-ionic surfactants, preferably in amounts of 0 to 30 wt.%; and/or
(iii) optional electrolytes, particularly inorganic and/or organic salts, particularly phosphate, citrate and/or sulfate, particularly preferably sodium sulfate, preferably in amounts of 5 to 30 wt.%; and/or
(iv) optional chelating agents, particularly selected from the group of quinoline and/or its salts, alkali metal polyphosphonates, picolinic acid und dipicolinic acid, mono- or polyphosphonic acids, particularly 1-hydroxyethylidene-1,1-diphosphonic acid (HEDP), ethylene diamine tetraacetic acid (EDTA), diethylene triamine penta(methylenephosphonic acid) (DTPMP), azacycloheptane diphosphonate (AHP), nitrilotriacetic acid (NTA), citrate and/or short chain dicarboxylic acids, preferably in amounts of 0 to 10 wt.%; and/or
(v) optional enzymes, such as proteases, amylases, cellulases and/or lipases, and/or enzyme stabilizers, preferably in amounts of 0 to 10 wt.%; and/or
(vi) optional builders, particularly fatty acids, preferably saturated and/or branched fatty acids, particularly with a melting point below 30 °C, and/or citric acid and/or citrate, preferably in amounts of 0 to 15 wt.%; and/or
(vii) optional fragrances, preferably in amounts of 0 to 5 wt.%; and/or
(viii) optional auxiliaries, such as defoamers, pH regulators, rheology modifiers (thickeners), solvents, colorants; and/or
(ix) optional additional usual ingredients, such as brighteners; and/or
(x) water;
wherein all the specified weights are based on the washing or cleansing agent.

10. Process for stabilizing percarboxylic acids, particularly imidopercarboxylic acids, which are solid at room temperature, in dispersions containing surfactants, preferably aqueous dispersions, wherein
- the total content of halide ions, particularly chloride and/or bromide, based on the continuous dispersion phase, is adjusted to not exceed 100 ppm, particularly not more than 50 ppm, preferably not more than 30 ppm, particularly preferably not more than 15 ppm; and/or
- the dispersion is adjusted to a pH of maximum 7, particularly a pH of 3.5 to 7, preferably from 4.0 to 6.5, particularly preferably from 4.5 to 6, quite particularly preferably to about 5; and/or
- the total active surfactant content in the dispersion or the continuous phase of the dispersion is adjusted to less than 5 %, particularly less than 2.5 %, preferably less than 1 %, based on the continuous dispersion phase.

## Revendications

1. Dispersion aqueuse contenant un/des agents tensioactifs, stable à l'entreposage d'acides peroxycarboxyliques solides à température ambiante, sous forme de particules, en particulier d'acides imidoperoxycarboxyliques, **caractérisée en ce que** dans la dispersion, la teneur totale en ions halogénure, en particulier chlorure et/ou bromure, par rapport à la phase continue de la dispersion, ne dépasse pas 100 ppm, en particulier pas 50 ppm, de préférence pas 30 ppm, de manière particulièrement préférée pas 15 ppm ; **en ce que** la dispersion présente un pH d'au maximum 7, en particulier un pH dans la plage de 3,5 à 7, de préférence de 4,0 à 6,5, de manière particulièrement préférée de 4,5 à 6, de manière tout particulièrement préférée d'environ 5 ; et **en ce que** la dispersion ne contient pas, du moins essentiellement, d'agents tensioactifs sous forme active, ou la teneur totale en agents tensioactifs actifs dans la phase continue de la dispersion est inférieure à 5%, en particulier inférieure à 2,5%, de préférence inférieure à 1%, à chaque fois par rapport à la phase continue de la dispersion.

2. Dispersion selon la revendication 1, **caractérisée en ce que** la dispersion contient au moins un complexant et/ou **en ce que** la dispersion contient au moins une catalase et/ou au moins une peroxydase, de préférence une catalase et/ou au moins un antioxydant et/ou **en ce que** la dispersion contient au moins un solvant en particulier miscible à l'eau avec un pouvoir de solubilisation faible pour les acides peroxycarboxyliques organiques, en particulier les acides imidoperoxycarboxyliques, de préférence le glycérol.

3. Dispersion selon la revendication 1 ou 2, **caractérisée en ce que** les acides peroxycarboxyliques sont en outre pourvus d'une enveloppe ou incorporés dans au moins une matrice.

4. Dispersion selon l'une quelconque des revendications 1 à 3, **caractérisée en ce que** la grosseur des particules des acides peroxycarboxyliques organiques utilisés est ≤ 3000 µm, en particulier ≤ 2500 µm, de préférence ≤ 2250 µm, de préférence ≤ 2000 µm, de manière particulièrement préférée ≤ 1500 µm et/ou **en ce que** la grosseur des particules des acides peroxycarboxyliques organiques utilisés est de 10 à 3000 µm, en particulier de 50 à 2500 µm, de préférence de 100 à 1500 µm.

5. Dispersion selon l'une quelconque des revendications 1 à 4, **caractérisée en ce que** la teneur en acides peroxycarboxyliques organiques, en particulier en acides imidoperoxycarboxyliques, par rapport à la dispersion, est de 0,1 à 30% en poids, en particulier de 0,5 à 25% en poids, de préférence de 1 à 20% en poids, de préférence de 1 à 15% en poids.

6. Dispersion selon l'une quelconque des revendications 1 à 5, **caractérisée en ce que** les acides peroxycarboxyliques organiques sont choisis parmi les acides monoperoxycarboxyliques et diperoxycarboxyliques organiques, en particulier le dodécanediperoxyacide ou de préférence les acides imidoperoxycarboxyliques, de manière particulièrement préférée l'acide 6-phtalimidoperoxycaproïque (acide 6-phtalimidoperoxyhexanoïque, PAP), et/ou **en ce que** les acides peroxycarboxyliques présentent, à pression atmosphérique, un point de fusion supérieur à 20°C, en particulier supérieur à 25°C, de préférence supérieur à 35°C, de préférence supérieur à 45°C, de manière particulièrement préférée supérieur à 50°C, de manière tout particulièrement préférée supérieur à 100°C.

7. Utilisation des dispersions selon l'une quelconque des revendications 1 à 6 dans ou comme agent(s) de lavage et de nettoyage, en particulier comme composition(s) liquide(s) d'agent de lavage et de nettoyage, agent(s) de soins dentaires, agent(s) de teinture des cheveux ou compositions d'agents de décoloration ou, selon le cas, de blanchiment destinées à des utilisations techniques.

8. Agent de lavage et de nettoyage, en particulier composition liquide d'agent de lavage et de nettoyage, agent de soins dentaires, agent de teinture des cheveux ou agent de décoloration ou, selon le cas, de blanchiment destiné à des utilisations techniques, contenant une dispersion selon l'une quelconque des revendications 1 à 6.

9. Agent de lavage ou de nettoyage selon la revendication 8, contenant :
(i) au moins un acide peroxycarboxylique organique, en particulier un acide imidoperoxycarboxylique, en des quantités de 0,1 à 30% en poids, en particulier de 0,5 à 25% en poids, de préférence de 1 à 20% en poids, de préférence de 1 à 15% en poids ; et/ou
(ii) des agents tensioactifs, de préférence sous forme inactivée, en particulier des agents tensioactifs cationiques et/ou anioniques, de préférence en des quantités de 0 à 30% en poids, et/ou des agents tensioactifs non ioniques, de préférence en des quantités de 0 à 30% en poids ; et/ou
(iii) le cas échéant des électrolytes, en particulier des sels inorganiques et/ou organiques, en particulier le phosphate, le citrate et/ou le sulfate, de manière particulièrement préférée le sulfate de sodium, de préférence en des quantités de 5 à 30% en poids ; et/ou
(iv) le cas échéant des complexants, en particulier choisis parmi le groupe de la quinoléine et/ou de ses sels, les polyphosphonates de métal alcalin, l'acide picolinique et l'acide dipicolinique, les acides monophosphoniques ou polyphosphoniques, en particulier l'acide 1-hydroxyéthylidène-1,1-diphosphonique (HEDP), l'acide éthylènediaminetétraacétique (EDTA), l'acide diéthylènetriaminepenta(méthylènephosphonique) (DTPMP), l'azacycloheptanediphosphonate (AHP), l'acide nitrilotriacétique (NTA), le citrate et/ou les acides dicarboxyliques à courte chaîne, de préférence en des quantités de 0 à 10% en poids ; et/ou
(v) les cas échéant des enzymes, telles que des protéases, des amylases, des cellulases et/ou des lipases, et/ou des stabilisateurs d'enzymes, de préférence en des quantités de 0 à 10% en poids ; et/ou
(vi) le cas échéant des builders, en particulier des acides gras, de préférence des acides gras saturés et/ou ramifiés, présentant en particulier un point de fusion inférieur à 30°C, et/ou l'acide citrique et/ou le citrate, de préférence en des quantités de 0 à 15% en poids ; et/ou
(vii) le cas échéant des substances odoriférantes, de préférence en des quantités de 0 à 5% en poids ; et/ou
(viii) le cas échéant des adjuvants, tels que des antimousses, des régulateurs de pH, des agents de modification de la rhéologie (épaississants), des solvants, des colorants ; et/ou
(ix) le cas échéant d'autres constituants usuels, tels que des azurants optiques et/ou
(x) de l'eau ;
toutes les indications de poids se rapportant à l'agent de lavage ou de nettoyage.

10. Procédé pour stabilisation d'acides peroxycarboxyliques solides à température ambiante, en particulier les acides imidoperoxycarboxyliques dans des dispersions contenant un/des agents tensioactifs, en particulier aqueuses, **caractérisé en ce qu'**on règle
- la teneur totale en ions halogénure, en particulier chlorure et/ou bromure, par rapport à la phase continue de la dispersion, à des quantités qui ne dépassent pas 100 ppm, en particulier pas 50 ppm, de préférence pas 30 ppm, de manière particulièrement préférée pas 15 ppm ;
- le pH de la dispersion au maximum à 7, en particulier dans la plage de 3,5 à 7, de préférence de 4,0 à 6,5, de manière particulièrement préférée de 4,5 à 6, de manière tout particulièrement préférée d'environ 5 ; et
- la teneur totale en agents tensioactifs actifs dans la phase continue de la dispersion à moins de 5%, en particulier moins de 2,5%, de préférence moins de 1%, à chaque fois par rapport à la phase continue de la dispersion.
